# EUROPÄISCHE PATENTANMELDUNG

(11) **EP 4 368 139 A1**
(43) Veröffentlichungstag der Anmeldung: **15.05.2024**
(21) Anmeldenummer: 23208026.7
(22) Anmeldetag: 06.11.2023
(51) Int. Cl.: A61B 34/20, A61B 34/10

(54) **KAVITÄTSMODELLIERUNGSSYSTEM UND KAVITÄTSMODELLIERUNGSVERFAHREN**

(30) Priorität: 14.11.2022 DE 102022130075
(71) Anmelder: B. Braun New Ventures GmbH, 79110 Freiburg im Breisgau (DE)
(72) Erfinder: SARVESTANI, Amir, 79104 Freiburg (DE)
(74) Vertreter: Winter, Brandl - Partnerschaft mbB

(57) **Zusammenfassung**

Die Offenbarung betrifft ein Kavitätsmodellierungssystem (1) für eine intraoperative Erstellung eines 3D-Modells (2) einer Kavität (K) in einem Patienten (P) bei einem chirurgischen Eingriff mit: einer Visualisierungseinheit (4) mit einem distalen Aufnahmekopf (6), die dafür angepasst ist intrakorporal in den Patienten (P) eingeführt zu werden und über den distalen Aufnahmekopf (6) eine intrakorporale Aufnahme (8) von zumindest einem Teilbereich der Kavität (K) des Patienten (P) zu erstellen und digital bereitzustellen, insbesondere einem Endoskop (10) mit einem distalen optischen System (12) und einem nachgeschalteten Aufnahmesensor (14) zur Erstellung der intrakorporalen Aufnahme (8), ferner aufweisend eine 3D-Modellierungseinheit (16), die dafür angepasst ist, ein digitales 3D-Modell (2) einer Innenoberfläche (18) der Kavität (K) zu erstellen und dieses um eine bereitgestellte erste Aufnahme (8a) in einer ersten Aufnahmelage und um zumindest eine zweite Aufnahme (8b) in einer zweiten Aufnahmelage zu ergänzen und anzupassen, wobei die 3D-Modellierungseinheit (16) ferner dafür angepasst ist, eine Ansicht des erstellten 3D-Modells (2) der Kavität (K) über eine visuelle Darstellungsvorrichtung (20), wie einem Display oder einer Virtual-Reality-Brille, auszugeben, um einem Nutzer wie etwa einem medizinischen Fachpersonal, eine zeitaktuelle intraoperative Visualisierung der Kavität (K) bereitzustellen. Daneben betritt die Offenbarung ein Kavitätsmodellierungsverfahren, ein computerlesbares Speichermedium sowie ein Computerprogramm gemäß den nebengeordneten Ansprüchen.

## Beschreibung

### Technisches Gebiet

Die vorliegende Offenbarung betrifft ein Kavitätsmodellierungssystem/ Kavitätsscanner für eine intraoperative Erstellung eines (digitalen und dadurch auch visuellen bzw. visualisierbaren) 3D-Modells einer Kavität/eines Hohlraums in einem Patienten bei einem chirurgischen Eingriff, insbesondere bei einer Gehirnoperation mit Tumorentfernung, mit: einer Visualisierungseinheit oder einem Visualisierungssystem mit einem distalen (bzw. endständigen) Aufnahmekopf, die dafür angepasst ist (insbesondere über seine Konfiguration mit entsprechender Geometrie und Abmessungen) in eine Öffnung (z.B. Einstichstelle oder Einschnitt) des Patienten (zumindest abschnittsweise) eingeführt zu werden und über den (distalen) Aufnahmekopf eine intrakorporale Aufnahme von (zumindest einem Teilabschnitt) einer Kavität des Patienten zu erstellen und digital /computerlesbar bereitzustellen, insbesondere einem 2D-Endoskop mit einem optischen System und einem nachgeschalteten Aufnahmesensor zur Erstellung einer intrakorporalen zweidimensionalen Aufnahme. Daneben betrifft die vorliegende Offenbarung ein Kavitätsmodellierungsverfahren, ein computerlesbares Speichermedium sowie ein Computerprogramm gemäß den Oberbegriffen der nebengeordneten Ansprüche.

### Hintergrund der Offenbarung

In der Tumorchirurgie im Bereich des Gehirns (Neurochirurgie) besteht das Hauptziel darin, den Tumor präzise zu entfernen und dabei einerseits das gesamte pathologische Gewebe zu resezieren und andererseits die Entfernung von gesundem Gewebe zu vermeiden. Zu diesem Zweck wird der Tumor oft von innen nach außen entfernt, wodurch ein Hohlraum/eine Kavität im Gehirn entsteht. Aufgrund des eingeschränkten Zugangs, insbesondere bei tiefsitzenden Tumoren, hat der Chirurg oft keine genauen Informationen über die aktuelle Kavität mit entsprechender Form des Hohlraums, die er mit einem präoperativen Plan aus einer Magnetresonanztomografie-Aufnahme (MRT-Scan/ MR-Scan) oder einer Computertomografie-Aufnahme (einem CT-Scan) vergleichen könnte, um sicherzustellen, dass der Tumor auch tatsächlich vollständig entfernt wurde. Solche neurochirurgischen Eingriffe werden insbesondere mit einem Operationsmikroskop durchgeführt, mit dem jedoch nicht die gesamte Kavität erfasst werden kann, vor allem nicht die Teilbereiche, die keine Sichtlinie bzw. keinen Sichtbereich für die optische Achse des Operationsmikroskops haben.

Um diese Beschränkung der Sicht in einen Innenraum bzw. Hohlraum zu überwinden, werden manchmal parallel zu dem Operationsmikroskop auch chirurgische Endoskope verwendet, um die Resektionshöhle bzw. den Hohlraum vom Inneren des Patienten aus zu betrachten. Auf diese Weise kann der Chirurg zwar mit manueller Führung nach und nach Teilbereiche und damit gewissermaßen die gesamte Kavität erfassen (in Art einer zeitaktuellen Videoaufnahme), die einzelnen Aufnahmen sind jedoch nur Ausschnitte der gesamten Kavität und geben dem Chirurgen kein geeignetes Modell der Kavität zur Hand, um einen Eingriff erfolgreich und sicher durchzuführen und zudem noch zu verifizieren.

3D-Scanner bzw. 3D-Modellierungssysteme für eine Erfassung einer Außenoberfläche eines Objekts sind in der zahnärztlichen Behandlung bekannt, um ein 3D-Modell einer Zahnstruktur zu erstellen. Diese 3D-Scanner arbeiten mit verschiedenen Technologien wie etwa mit 3D-Laserscannern oder 3D-Punktwolkenkameras. Im Bereich der Neurochirurgie lassen sich solche Scanner aufgrund der geringen Größe der (Einstichs-)Schnitte und der Tumorhöhle jedoch nicht einsetzen. Zudem lassen sich mit solchen dentalen Scannern keine Kavitäten abtasten und vermessen.

### Zusammenfassung der Offenbarung

Die Aufgabe der vorliegenden Offenbarung ist daher, die Nachteile aus dem Stand der Technik zu vermeiden oder zumindest zu mindern und insbesondere ein Kavitätsmodellierungssystem/ Kavitätsscanner, ein Kavitätsmodellierungsverfahren, ein computerlesbares Speichermedium sowie ein Computerprogramm zur Verfügung zu stellen, welches es einem Nutzer erlauben bei einem chirurgischen Eingriff mit einem Visualisierungssystem wie etwa einem Endoskop, insbesondere einem Neuroendoskop, eine Kavität/ eine Resektionshöhle/ einen Resektionshohlraum intraoperativ (also während der Operation) zu erfassen und auf Basis der intraoperativen Erfassung auch intraoperativ ein dreidimensionales Modell (3D-Modell) dieser Kavität (zumindest teilbereichsweise, insbesondere der gesamten Kavität) zu erstellen. Eine weitere Teilaufgabe kann darin gesehen werden, dem Nutzer eine intuitive Möglichkeit einer Erfassung zu bieten, mit dem er eine geführte Erfassung der Kavität/ des Resektionshohlraums, insbesondere der gesamten Kavität, bewerkstelligen kann. Eine weitere Teilaufgabe besteht darin, bei einem robotergeführten Visualisierungssystem eine Modalität einer automatischen oder einer manuellen Steuerung oder eine Kombination aus einer manuellen und automatischen Steuerung bereitzustellen, mit welcher der Nutzer noch einfacher und intuitiver die Kavität abfahren, abtasten/abscannen und erfassen kann.

Die Aufgaben der vorliegenden Offenbarung werden hinsichtlich eines Kavitätsmodellierungssystem/ Kavitätsscanner erfindungsgemäß durch die Merkmale des Anspruchs 1 gelöst, hinsichtlich eines Kavitätsmodellierungsverfahrens erfindungsgemäß durch die Merkmale des Anspruchs 12 gelöst, hinsichtlich eines computerlesbaren Speichermediums erfindungsgemäß durch die Merkmale des Anspruchs 14 gelöst und hinsichtlich eines Computerprogramms erfindungsgemäß durch die Merkmale des Anspruchs 15 gelöst.

Ein Grundgedanke der vorliegenden Offenbarung sieht also vor, ein System zur Erstellung eines dreidimensionalen (Innen-)Oberflächenmodells (3D-Modells) eines Hohlraums bereitzustellen, welches während eines chirurgischen Eingriffs wie etwa einem neurochirurgischen Eingriff am Gehirn (einer Gehirnoperation) während einer Tumorresektion, das 3D-Modell erstellt, insbesondere unter Verwendung von zweidimensionalen Bildern (2D-Bildern) einer bewegten Visualisierungseinheit wie einem Endoskops, also eines Endoskops, dessen optische Achse des Aufnahmekopfs sich in der Kavität bewegt. Damit kann eine Technologie bereitgestellt werden, bei der insbesondere ein 2D-Endoskop zur Erstellung eines 3D-Modells der Tumorhöhle in der Neurochirurgie verwendet wird. Die Kavität bzw. der Hohlraum befindet sich beispielsweise im Falle der Neurochirurgie innerhalb des Gehirns.

Insbesondere kann also das Kavitätsmodellierungssystem/ der Kavitätsscanner (dafür angepasst sein) sogar nur mit Hilfe eines (herkömmlichen) 2D-Endoskops ein 3D-Modell der Operationshöhle erstellen. Hierfür wird insbesondere nach einer anfänglichen Resektion des Tumors das Endoskop in die Resektionshöhle/ Kavität eingeführt. Die Optik des Endoskops zeigt nur einen Ausschnitt der Kavität und nur als zweidimensionale Aufnahme (2D-Bild) an bzw. erfasst diese. Wenn das Endoskop nun so bewegt wird, dass insbesondere alle Bereiche der Kavität erfasst und abgebildet werden, kann ein (vollständiges) 3D-Modell der Resektionshöhle erstellt werden. Dieses visualisierte 3D-Modell kann der Chirurg wiederum für seine Bewertung und Anpassung seiner Tumorentfernung heranziehen.

Mit noch ganz anderen Worten wird ein Kavitätsmodellierungssystem mit einer Visualisierungseinheit (einem Visualisierungssystem) bereitgestellt, das (dafür angepasst ist, dass es) in eine chirurgische Kavität des Patienten einführbar ist, wobei das Visualisierungssystem insbesondere lediglich 2D-Bilder von verschiedenen Abschnitten der Kavität erstellt und das Kavitätsmodellierungssystem auf Basis der 2D-Bilder dann ein 3D-Modell der Kavität erstellt.

Noch anders ausgedrückt wird ein Kavitätsmodellierungssystem für eine intraoperative Erstellung eines 3D-(Oberflächen-)Modells einer Kavität in einem Patienten bei einem chirurgischen Eingriff, insbesondere bei einer Gehirnoperation mit Tumorentfernung, bereitgestellt. Dieses Kavitätsmodellierungssystem hat eine Visualisierungseinheit mit (einem proximalen Handhabungsabschnitt für eine manuelle Führung oder eine Anbindung an einen Roboter für eine Roboterführung) und einem distalen Aufnahmekopf, die dafür angepasst ist intrakorporal in den Patienten eingeführt zu werden und über den Aufnahmekopf eine intrakorporale Aufnahme von zumindest einem Teilbereich der Kavität des Patienten zu erstellen und digital/computerlesbar bereitzustellen, insbesondere in Form eines Endoskops mit einem optischen System und einem nachgeschalteten Aufnahmesensor zur Erstellung einer intrakorporalen Aufnahme. Ferner hat das Kavitätsmodellierungssystem eine 3D-Modellierungseinheit, die dafür angepasst ist, ein digitales 3D-(Hohlraumoberflächen-)Modell einer Innenoberfläche einer Kavität zu erstellen und dieses um die bereitgestellte erste Aufnahme in einer ersten Aufnahmelage und um zumindest eine zweite Aufnahme in einer zweiten Aufnahmelage zu ergänzen und anzupassen, wobei die 3D-Modellierungseinheit ferner dafür angepasst ist, eine Ansicht des erstellten 3D-Modells der Kavität über eine visuelle Darstellungsvorrichtung auszugeben, um einem Nutzer wie etwa einem medizinischen Fachpersonal, eine zeitaktuelle intraoperative Visualisierung der Kavität bereitzustellen. Das virtuelle 3D-(Hohlraumoberflächen-)Modell wird also insbesondere sukzessive um die entsprechenden Aufnahmen (lagekorrekt) ergänzt (ähnlich einer sukzessiven Panoramaaufnahme) bzw. falls schon ein initiales 3D-Modell vorliegt, entsprechend um die Aufnahmen in Referenz auf die Aufnahmelagen angepasst.

Der Begriff "Position" meint eine geometrische Position im dreidimensionalen Raum, der insbesondere mittels Koordinaten eines kartesischen Koordinatensystems angegeben wird. Insbesondere kann die Position durch die drei Koordinaten X, Y und Z angegeben werden.

Der Begriff "Orientierung" wiederum gibt eine Ausrichtung (etwa an der Position) im Raum an. Man kann auch sagen, dass durch die Orientierung eine Ausrichtung angegeben wird mit Richtungs- bzw. Drehungsangabe im dreidimensionalen Raum. Insbesondere kann die Orientierung mittels drei Winkeln angegeben werden.

Der Begriff "Lage" umfasst sowohl eine Position als auch eine Orientierung. Insbesondere kann die Lage mittels sechs Koordinaten angegeben werden, drei Positionskoordinaten X, Y und Z sowie drei Winkelkoordinaten für die Orientierung.

Der Begriff "3D" definiert dabei, dass die Aufnahmedaten räumlich, also dreidimensional vorliegen. Die Kavität des Patienten oder zumindest ein Teilbereich der Kavität mit räumlicher Ausdehnung kann in einem dreidimensionalen Raum mit etwa einem kartesischen Koordinatensystem (X, Y, Z) digital vorliegen. Insbesondere liegt ein 3D-Oberflächenmodell vor, also insbesondere eine geschlossene Oberfläche im Raum.

Der Begriff "2D" definiert dabei, dass die Aufnahmedaten zweidimensional vorliegen.

Vorteilhafte Ausführungsformen sind in den Unteransprüchen beansprucht und werden insbesondere nachfolgend erläutert.

Im Falle eines Endoskops als Visualisierungseinheit kann dieses insbesondere eine abgewinkelte Optik, etwa (eine optische Achse) um 60 Grad zu einer Längsachse des Endoskops, aufweisen. Durch eine Rotations- und/oder einer Axialbewegung der Endoskopachse kann somit auch die optische Achse bewegt werden und zumindest ein Teilbereich, insbesondere die gesamte Kavität erfasst und aufgenommen werden.

Gemäß einer Ausführungsform kann das Kavitätsmodellierungssystem ein Trackingsystem aufweisen, das dafür angepasst ist, eine Position und Orientierung (Lage) des Aufnahmekopfs der Visualisierungseinheit (direkt oder indirekt) im Raum nachzuverfolgen, um so die Aufnahmelage der Aufnahme zu bestimmen. Mit anderen Worten kann die Visualisierungseinheit / das Visualisierungssystem ein Trackingsystem/ Verfolgungssystem umfassen, das dafür vorgesehen und angepasst ist, die Position und/oder Orientierung (insbesondere die Lage) der Visualisierungseinheit und damit die Aufnahmelage im Raum zu bestimmen. Insbesondere kann eine Transformation von einem nachverfolgten Handhabungsabschnitt zu dem Aufnahmekopf, insbesondere in Form einer Kamera mit einer optischen Achse, bekannt sein, so dass über die Erfassung der Lage des Handhabungsabschnitts auf die Lage des Aufnahmekopfs und ferner, etwa über eine Bildanalyse oder über einen Entfernungssensor zur dreidimensionalen Erfassung einer Oberfläche, von der Lage des Aufnahmekopfs auf die Aufnahmelage geschlossen werden kann und diese entsprechend bestimmbar ist und die Aufnahme zusammen mit der Aufnahmelage bereitgestellt wird und entsprechend in dem 3D-Modell ergänzt oder angepasst wird.

Vorzugsweise kann die 3D-Modellierungseinheit dafür angepasst sein durch eine Bildanalyse der zweidimensionalen Aufnahme (2D-Scans), bei der die Bilder für verschiedene Positionen verglichen werden, ein 3D-Modell einer Innen-Oberfläche der abgebildeten Region und damit der Kavität zu erstellen. Bei einer Aufnahme der gesamten Kavität wird ein vollständiges 3D-Modell der Kavität erstellt. Alternativ oder zusätzlich zu der Bildanalyse kann die Bewegung des Endoskops auch mit einem Tracking-System verfolgt werden, um für jedes Bild die Position im Raum zu bestimmen und so ein 3D-Modell der Kavität zu erstellen.

Gemäß einer weiteren Ausführungsform kann das Trackingsystem ein Navigationssystem mit einer externen Navigationskamera aufweisen und/oder ein elektromagnetisches Navigationssystem aufweisen und/oder eine an der Visualisierungseinheit angeordnete Inertiale Messeinheit (inertialbasiertes Navigationssystem/ IMU-Sensor) aufweisen und/oder das Trackingsystem auf Basis einer Roboterkinematik eines chirurgischen Roboters mit der Visualisierungseinheit als Endeffektor die Lage des Aufnahmekopfs bestimmen. Mit anderen Worten kann das Tracking/ die Verfolgung der Visualisierungseinheit, insbesondere des Endoskops, insbesondere mit einem externen optischen und/oder elektromagnetischen Navigationssystem und/oder mit einem (für die Visualisierungseinheit internen) inertialbasierten Navigationssystem (etwa einen IMU-Sensor in der Visualisierungseinheit, insbesondere im Endoskop) und/oder unter Verwendung der Kinematik eines Roboterarms, der die Visualisierungseinheit (etwa das Endoskop) bewegt, realisiert werden. Insbesondere kann die Position und Ausrichtung der Visualisierungseinheit durch die Kinematik eines Roboterarms bestimmt werden, der die Bewegung ausführt.

Vorzugsweise kann die 3D-Modellierungseinheit dafür angepasst sein, mittels einer Bildanalyse auf Basis der ersten Aufnahme mit der ersten Aufnahmelage und der zumindest zweiten Aufnahme mit der zweiten Aufnahmelage eine dreidimensionale Innenoberfläche eines Bereichs der Kavität oder der gesamten Kavität zu bestimmen/ berechnen. Insbesondere kann also das System eine Datenverarbeitungseinheit (wie einen Computer) und eine Speichereinheit sowie spezielle Algorithmen zur Analyse der Aufnahmen/ Bilder aus verschiedenen Positionen umfassen, um eine 3D-Oberfläche entweder eines Bereichs des Hohlraums oder des gesamten Hohlraums zu erstellen.

Insbesondere kann die Visualisierungseinheit, insbesondere das Endoskop, eine Fluoreszenzaufnahmeeinheit mit einem Strahler einer vordefinierten Wellenlänge für eine Anregung und mit einem Sensor für eine Detektion aufweisen, wobei insbesondere über den Aufnahmekopf mit Aufnahmesensor eine Fluoreszenz detektierbar ist, um das 3D-Modell der Innenoberfläche der Kavität um weitere Annotationen, insbesondere zu einer Annotationen zu einer Tumoraktivität und/oder einem Blutfluss zu ergänzen, und zeitaktuelle und für den Eingriff relevante Informationen zur Verfügung zu stellen. Anders ausgedrückt kann die Visualisierungseinheit, insbesondere ein Endoskop mit einer integrierten Fluoreszenzbildgebung sein, das das 3D-Modell (die 3D-Hohlraumoberfläche) mit funktionellen Informationen, insbesondere mit Annotationen zu einer Tumoraktivität und/oder einem Blutfluss ergänzt, um den Nutzer weitere, zeitaktuelle und für den Eingriff relevante Informationen zur Verfügung zu stellen.

Gemäß einer Ausführungsform können in einer Speichereinheit des Kavitätsmodellierungssystems präoperative dreidimensionale Aufnahmen gespeichert sein, insbesondere MRT-Aufnahmen und/oder CT-Aufnahmen, die zumindest den Eingriffsbereich mit einem zu resezierenden Gewebe, insbesondere Tumor, umfassen, und das Kavitätsmodellierungssystem ferner eine Vergleichseinheit aufweisen, die dafür angepasst ist, das intraoperativ erstellte 3D-Modell der Innenoberfläche der Kavität mit einem dreidimensionalen Außenoberflächenmodell des zu entfernenden Gewebes, insbesondere Tumors, der präoperativen Aufnahme zu vergleichen und den Vergleich über die Darstellungsvorrichtung auszugeben, insbesondere eine Abweichung auszugeben, besonders bevorzugt in Form einer Überlagerungsdarstellung von dem intraoperativen 3D-Modell und dem präoperativen dreidimensionalen Oberflächenmodell und/oder einer Angabe einer prozentualen Abweichung eines Volumens, so dass dem Nutzer Bereiche einer Unter- oder Überresektion angezeigt werden. Insbesondere kann also das Kavitätsmodellierungssystem dafür angepasst sein, das 3D-Modell, insbesondere die 3D-Oberfläche des Hohlraums, mit dem 3D-(Oberflächen-)Modell des Tumors aus einem präoperativen 3D-Aufnahmen wie einem einer Magnetresonanztomografie (MRT) oder einer Computertomografie (CT) zu vergleichen. Ferner kann vorzugsweise das System Abweichungen zwischen der intraoperativ ermittelten 3D-Modell der Kavität und dem 3D-Modell des Tumors aus einem präoperativen Bild wie MR oder CT angeben, insbesondere anzeigen, so dass der Benutzer Bereiche mit Unter- oder Überresektion erkennen kann.

Ferner vorzugsweise kann die Vergleichseinheit dafür angepasst sein, eine dreidimensionale Form des intraoperativen 3D-Modells und des präoperativen dreidimensionalen Oberflächenmodells zu vergleichen, insbesondere ein Verhältnis einer Breite zu einer Länge zu einer Höhe zu vergleichen und über die Darstellungsvorrichtung auszugeben, insbesondere hinsichtlich einer Abweichung, um über den Formvergleich bei einer sich durch weiches Gewebe veränderlichen Kavität die Resektion zu veranschaulichen und insbesondere eine korrekte Resektion zu bestätigen.

Vorzugsweise kann das Kavitätsmodellierungssystem über die Darstellungsvorrichtung zeitaktuell eine Ansicht des 3D-Modells mit erfassten Bereichen mit den intraoperativen Aufnahmen anzeigen und ebenso mit den Bereichen anzeigen, die noch nicht erfasst sind und bei einer manuellen Führung der Visualisierungseinheit, insbesondere des Endoskops, eine Anleitung für eine Erfassung der noch zu erfassenden Bereichen an den Nutzer auszugeben, insbesondere in Form von Pfeilen, welche eine Translationsrichtung und/oder Rotationsrichtung vorgeben, um dem Nutzer eine intuitive, vollständige Erfassung der Kavität bereitzustellen.

Vorzugsweise kann eine Bewegung der Visualisierungseinheit entweder manuell durch einen Chirurgen durchgeführt werden oder automatisch durch einen Roboterarm oder durch eine Kombination aus beidem. Die Visualisierungseinheit kann also insbesondere manuell oder durch einen Roboter oder auch durch eine Kombination bewegt werden. Das Visualisierungssystem kann beispielsweise von einem Nutzer nur manuell bewegt, um die Oberfläche des Hohlraums zu erfassen/ abzutasten. Die Visualisierungseinheit kann insbesondere auch von einem bzw. durch einen Roboter bewegt werden. Dieser Roboter kann insbesondere vollständig vom Benutzer manuell gesteuert werden oder sich autonom bewegen (etwa nach einem automatischen Steuerverfahren zur Erfassung der Kavität), um die Oberfläche des Hohlraums zu scannen.

Vorzugsweise kann das Kavitätsmodellierungssystem einen Roboter mit einem Roboterarm aufweisen, an dem die Visualisierungseinheit, insbesondere das Endoskop, als Endeffektor angebunden ist, wobei eine Steuereinheit des Kavitätsmodellierungssystems dafür angepasst ist, den Roboter und damit die Lage des Aufnahmekopfs der Visualisierungseinheit zu steuern und insbesondere für eine Erfassung der Kavität diese automatisch abzufahren, insbesondere um die gesamte Innenoberfläche der Kavität zu erfassen.

Gemäß einer Ausführungsform kann die Steuereinheit dafür angepasst sein, die Position des Aufnahmekopfs mit drei Positionsparametern und die Orientierung des Aufnahmekopfs mit drei Orientierungsparametern zu steuern, wobei eine Teilmenge der (Steuer-)Parameter der automatischen Steuerung zugeordnet ist und durch die Steuereinheit automatisch ausgeführt wird und eine verbleibende Teilmenge der Parameter der manuellen Steuerung zugeordnet wird und über eine Eingabeeinheit durch den Nutzer steuerbar ist, wobei vorzugsweise eine Rotation des Aufnahmekopfs über die Orientierungsparameter der manuellen Steuerung zugeordnet wird und eine axiale, translatorische Bewegung über die Positionsparameter der automatischen Steuerung zugeordnet wird.

Die Visualisierungseinheit kann vorzugsweise auch in einer manuellautomatischen Steuerung bewegt werden, bei der die Visualisierungseinheit einerseits vom Nutzer manuell bewegt wird, insbesondere nur die Rotation durch den Nutzer manuell gesteuert wird, kombiniert mit einer autonomen Bewegung durch einen Roboter, insbesondere nur die axiale Bewegung. So kann beispielsweise durch den Nutzer die Rotation manuell gesteuert werden, während der Roboter eigenständig die axiale Bewegung automatisch durchführt, so dass eine kombinierte Bewegung der Visualisierungseinheit erfolgt, mit sowohl einer manuellen Steuerung von Parametern einer Rotation als auch einer automatischen Steuerung von Parametern einer Translation bzw. einer axialen Bewegung. Mit anderen Worten können die Parameter von Freiheitsgraden aufgeteilt werden, während eine Teilmenge der Parameter der manuellen Steuerung zugeordnet wird und ein anderer Teil der Parameter der automatischen Steuerung zugeordnet wird.

In einer Ausführungsform kann zunächst eine automatische Steuerung durch den Roboter erfolgen und bei Bedarf eine manuelle Steuerung die automatische Steuerung, teilweise hinsichtlich einer vordefinierten Teilmenge an Parameter an Freiheitsgraden oder auch vollständig, durch eine manuelle Steuerung abgelöst werden, so dass im Bedarfsfall ein automatisches Abfahren der Kavität durch eine manuelle Bewegung überschrieben und damit übernommen werden kann.

Insbesondere kann die Visualisierungseinheit in Form eines Endoskops, insbesondere eines 2D-Endoskops oder eines 3D-Endoskops, ausgebildet sein und eine Kamera aufweisen. Insbesondere kann deren optische Achse quer zu einer Längsachse des Endoskops ausgerichtet sein, insbesondere die optische Achse in eine radiale Außenseite des Endoskopschafts einstehen und das Endoskop weiter vorzugsweise eine Weitwinkelkamera an einer radialen Außenseite aufweisen, die einen Sehwinkel von über 60° erfasst, um mittels Rotation die Innenoberfläche der Kavität zu erfassen. Die Visualisierungseinheit kann also insbesondere in Form eines 2D-Endoskops ausgestaltet sein, welches zweidimensionale Aufnahmen erstellt (2D-Scans). Alternativ kann die Visualisierungseinheit ein 3D-Endoskop sein, welches dreidimensionale Aufnahmen (3D-Scans) erstellt.

Das Kavitätsmodellierungssystem kann vorzugsweise ein Display oder eine 3D-Brille (virtuell oder augmentiert) zur Ausgabe des 2D-Bildes während der Bewegung des Visualisierungssystems und der erzeugten 3D-Oberfläche an den Benutzer umfassen.

Vorzugsweise kann das 3D-(Hohlraum-)Modell verwendet werden, um intraoperativ eine Hirnverschiebungskorrektur vorzunehmen, wenn ein Navigationssystem verwendet wird, indem die präoperative 3D-Aufnahme mit dem 3D-Modell korrigiert wird.

Insbesondere kann das System dafür angepasst sein, das 3D-Modell mit zusätzlichen Informationen aus weiteren Modalitäten, insbesondere einem Neuro-Monitoring und/oder einer Histologie, zu ergänzen.

Insbesondere kann das Kavitätsmodellierungssystem dafür angepasst sein, eine prozentuale Anzeige einer (sphärischen) Erfassung der Kavität über die Darstellungsvorrichtung auszugeben und eine visuelle Anzeige von Bereich der Kavität ausgeben, die noch zu erfassen sind.

Insbesondere kann die Visualisierungseinheit ein starres Endoskop sein. Insbesondere kann die Visualisierungseinheit ein Neuroendoskop sein.

Insbesondere kann ein Durchmesser eines Endoskopschafts kleiner als 10mm, bevorzugt kleiner als 5mm sein. Insbesondere kann eine Abmessung eines Aufnahmekopfs kleiner als 5mm sein.

Insbesondere kann das Kavitätsmodellierungssystem dafür angepasst sein mittels einer Panoramafunktion aus bewegten Bildern das 3D-Modell zu erstellen.

Insbesondere kann das Kavitätsmodellierungssystem dafür angepasst sein, eine Vollständigkeit einer Erfassung der Resektionskavität(-höhle) zu überprüfen und in dem Fall, dass noch keine Vollständige Erfassung vorliegt eine Anweisung einer Bewegung der Visualisierungseinheit an einen Nutzer ausgeben und in dem Fall, dass eine vollständige Erfassung vorliegt, eine Ansicht des 3D-Modells oder einen Vergleich mit einem präoperativen dreidimensionalen Oberflächenmodell ausgeben. Wichtig ist vorliegend also ein Vollständigkeitsmerkmal der Erfassung der Innenoberfläche der Kavität, welches das Kavitätsmodellierungssystem bestimmen kann.

Insbesondere kann die Nachverfolgung/ Tracking bei einem starren Endoskop indirekt über ein Handstück oder angebrachten Tracker (mit Markern) sowie einer bekannten starren Transformation von Handstück bzw. Tracker zu Aufnahmekopf erfolgen.

Insbesondere können über die Darstellungsvorrichtung Bereiche, die erfasst sind, angezeigt werden und ebenso Bereichen, die nicht erfasst sind, angezeigt werden. Vorzugsweise kann das Kavitätsmodellierungssystem die Visualisierungseinheit zu den Bereichen führen, welche noch nicht erfasst sind.

Insbesondere kann das 3D-Modell auf eine Innenoberfläche eines Hohlraums angepasst sein. Insbesondere kann zunächst ein sphärisches Oberflächenmodell als Ausgangsmodell gewählte werden, welches dann durch die Aufnahmen um die Oberfläche in diesem Bereich entsprechend angepasst wird. Insbesondere kann also ein 3D-Oberflächen-Modell (mit einer Vollummantelung) über die zweidimensionalen Aufnahmen entsprechend angepasst.

Hinsichtlich eines Kavitätsmodellierungsverfahren für eine intraoperative Erstellung eines 3D-Modells einer Kavität in einem Patienten bei einem chirurgischen Eingriff, insbesondere bei einer Gehirnoperation mit Tumorentfernung Kavitätsmodellierungsverfahren werden die Aufgaben dadurch gelöst, dass dieses die Schritte aufweist: Vorzugsweise intrakorporales Einführen einer Visualisierungseinheit mit einem distalen Aufnahmekopf in den Patienten, insbesondere eines Endoskops mit einem optischen System und einem nachgeschalteten Aufnahmesensor zur Erstellung einer intrakorporalen Aufnahme; Erstellen einer ersten Aufnahme durch das Visualisierungssystem in einer ersten Aufnahmelage; Erstellen zumindest einer zweiten Aufnahme durch das Visualisierungssystem in einer zweiten Aufnahmelage, die unterschiedlich zu der ersten Aufnahmelage ist; Erstellen eines 3D-(Hohlraumoberflächen-)Modells einer Innenoberfläche einer Kavität und Ergänzen und Anpassen um die bereitgestellte erste Aufnahme in einer ersten Aufnahmelage und um zumindest eine zweite Aufnahme in einer zweiten Aufnahmelage; und Ausgeben einer Ansicht des erstellten 3D-Modells der Kavität über eine visuelle Darstellungsvorrichtung, um einem Nutzer wie etwa einem medizinischen Fachpersonal, eine zeitaktuelle intraoperative Visualisierung der Kavität bereitzustellen.

Insbesondere kann das Kavitätsmodellierungsverfahren ferner die Schritte aufweisen: Vergleichen einer Form eines präoperativen dreidimensionalen Oberflächenmodells mit dem 3D-Modell; und Ausgeben, durch die Darstellungsvorrichtung, einer Überlagerungsdarstellung, insbesondere einer jeweils teiltransparenten Ansicht, des intraoperativen 3D-Modells der Innenoberfläche der Kavität und des präoperativen dreidimensionalen Oberflächenmodells, um dem Nutzer eine Resektion zu veranschaulichen.

Hinsichtlich eines computerlesbares Speichermedium sowie eines Computerprogramms werden die Aufgaben dadurch gelöst, dass dieses Befehle umfasst, die, bei einer Ausführung durch einen Computer, diesen veranlassen, die Verfahrensschritte des Kavitätsmodellierungsverfahrens gemäß der vorliegenden Offenbarung auszuführen.

Jegliche Offenbarung im Zusammenhang mit dem Kavitätsmodellierungssystem gemäß der vorliegenden Offenbarung gilt ebenso (analog) auch für das Kavitätsmodellierungsverfahren gemäß der vorliegenden Offenbarung wie auch umgekehrt.

### Kurzbeschreibung der Figuren

Die vorliegende Offenbarung wird nachfolgend anhand bevorzugter Ausführungsformen unter Bezugnahme auf die begleitenden Figuren näher erläutert. Es zeigen:
Fig. 1 eine perspektivische Ansicht eines Kavitätsmodellierungssystems gemäß einer ersten bevorzugten Ausführungsform der vorliegenden Offenbarung,
Fig. 2 eine schematische Längsschnittansicht durch ein Gehirn eines Patienten mit Kavität, in welche ein Endoskop eingeführt ist, um die Kavität zu erfassen und ein 3D-Modell zu erstellen;
Fig. 3 eine perspektivische Ansicht einer weiteren Ausführungsform eines Kavitätsmodellierungssystems gemäß einer weiteren bevorzugten Ausführungsform, in welcher das Endoskop automatisch durch einen Roboter geführt ist um die Kavität zu erfassen;
Fig. 4 eine schematische Darstellung einer Bildverarbeitung, um zu veranschaulichen, wie mittels der Vielzahl an 2D-Aufnahmen ein 3D-Modell erstellt werden kann; und
Fig. 5 ein Flussdiagramm eines Kavitätsmodellierungsverfahrens gemäß einer ersten bevorzugten Ausführungsform.

Die Figuren sind schematischer Natur und sollen nur dem Verständnis der Erfindung dienen. Gleiche Elemente sind mit denselben Bezugszeichen versehen. Die Merkmale der verschiedenen Ausführungsbeispiele können untereinander ausgetauscht werden.

### Detaillierte Beschreibung bevorzugter Ausführungsformen

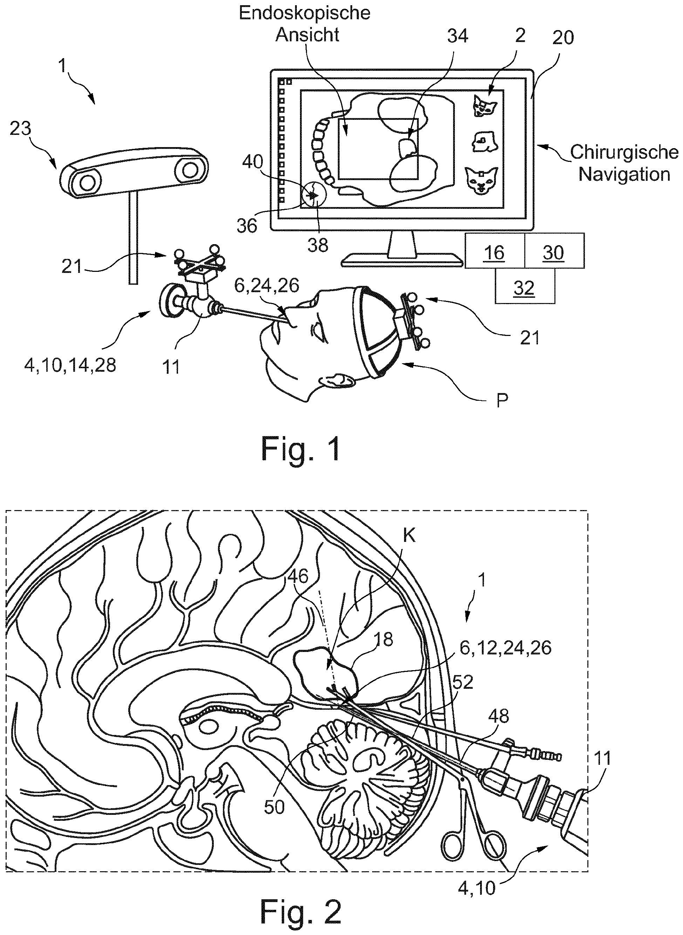

Fig. 1 zeigt in einer schematischen, perspektivischen Ansicht ein Kavitätsmodellierungssystem 1 gemäß einer ersten bevorzugten Ausführungsform der vorliegenden Offenbarung, welches bei einem neurochirurgischen Eingriff einer Tumorentfernung am Gehirn eines Patienten P eingesetzt wird.

Das Kavitätsmodellierungssystem 1 umfasst dabei eine Visualisierungseinheit 4 mit einem distalen Aufnahmekopf 6 in Form eines Endoskops 10 mit einem distalen optischen System 12 und einem nachgeschalteten Aufnahmesensor 14. Das Endoskop ist dafür angepasst intrakorporal in den Patienten P in das Gehirn selbst eingeführt zu werden und über den Aufnahmekopf 6 dann eine intrakorporale Aufnahme 8 von zumindest einem Teilbereich der Kavität K des Patienten P zu erstellen und digital bereitzustellen. Das vorliegende Kavitätsmodellierungssystem 1 weist dabei eine 3D-Modellierungseinheit 16 (mit einem Prozessor und einem Speicher) auf, die dafür angepasst ist, ein digitales 3D-Modell 2 einer Innenoberfläche 18 der Kavität K zu erstellen und dieses um eine bereitgestellte erste Aufnahme 8a in einer ersten Aufnahmelage und um zumindest eine zweite Aufnahme 8b in einer zweiten Aufnahmelage zu ergänzen und anzupassen. Das Endoskop 10 kann über seinen proximalen Handhabungsabschnitt 11 entweder manuell geführt oder an einen Roboter angebunden werden, um im Inneren der Resektionshöhle bzw. in der Kavität K des zu entfernenden Tumors bewegt zu werden. In dieser Ausführungsform wird das Endoskop 10 sogar kontinuierlich solange bewegt, bis Aufnahmen 8 von der gesamten Kavität K vorliegen, die in das 3D-Modell 2 eingebunden werden, um so ein komplettes 3D-Modell 2 der Innenoberfläche der Kavität K zu erstellen.

Die Aufnahmen 8 werden dabei hinsichtlich einer dreidimensionalen Innenoberfläche der Kavität K analysiert und das 3D-Modell 2 an den entsprechenden Bereichen hinsichtlich der berechneten dreidimensionalen Form erstellt bzw. angepasst. Zudem werden zusätzlich zu der geometrischen Formgebung des 3D-Modells 2 auch noch die (farbigen) Bilder eingebunden, so dass zusätzlich zu den räumlichen Informationen noch die Bildinformationen im 3D-Modell 2 eingebunden sind.

Die 3D-Modellierungseinheit 16 ist zudem dafür angepasst, eine Ansicht des erstellten 3D-Modells 2 der Kavität K über eine visuelle Darstellungsvorrichtung 20, in Form eines OP-Monitors auszugeben, um einem Nutzer wie etwa einem medizinischen Fachpersonal, eine zeitaktuelle intraoperative Visualisierung der Kavität K bereitzustellen. Ähnlich eines CAD-Modells, welches um verschiedene Achsen drehbar und im Raum verschiebbar ist, mit optionalen Möglichkeiten von Längsschnitten oder Querschnitten sowie Zoomfunktionen, um bestmögliche Ansichten zu generieren, kann eine Ansicht des digitalen 3D-Modells 2 über die Darstellungsvorrichtung ausgegeben werden, welche der Chirurg für seinen Eingriff verwendet.

Das Kavitätsmodellierungssystem 1 bzw. der Kavitätsscanner 1 scannt also eine Kavität K mit dem Endoskop 10 vollständig ab (im Unterschied zu einem dentalen 3D-Scanner etwa, der dafür angepasst ist ein Objekt mit einer Außenoberfläche zu erfassen) und erstellt ein digitales Oberflächen-Modell des gescannten Hohlraums, das Informationen zu einer geometrischen Form innehat. Mit Hilfe dieses 3D-Modells 2 kann der Chirurg dann erkennen, ob seine Resektion korrekt ist oder ob eine Über- oder Unterresektion vorliegt.

Ferner weist das Kavitätsmodellierungssystem 1 ein Trackingsystem 22 auf, das dafür angepasst ist, eine Position und Orientierung des distalen Aufnahmekopfs 6 der Endoskops 10 im Raum nachzuverfolgen, um so die Aufnahmelage der intrakorporalen Aufnahme 8 zu bestimmen. In dieser Ausführungsform ist das Trackingsystem in Form eines optischen Navigationssystems mit externen Tracker 21 in Form von Starrkörpern mit optischen Markern und einer externen Navigationskamera 23 (in Form einer stereoskopischen Kamera). Ein Tracker 21 ist dabei an dem Handhabungsabschnitt 11 des Endoskops 10 angebracht und zudem ist dem Navigationssystem eine starre Transformation von Lage Tracker zu Lage Frontlinse bekannt, so dass hierüber die Aufnahmelage bestimmbar ist. Ebenso ist an dem Patienten P an dem Kopf ein Tracker 21 befestigt, um auch den Kopf und damit den Eingriffsbereich mit der Kavität K durch die externe Navigationskamera 23 nachzuverfolgen.

Ferner hat das Endoskop an seinem distalen Ende eine Fluoreszenzaufnahmeeinheit 24 mit sowohl einem Strahler 26 (hier UV-Strahler) einer vordefinierten Wellenlänge (UV-Wellenlänge) für eine Anregung. Als Sensor 28 für eine Detektion der Fluoreszenz dient vorliegend der Aufnahmesensor 14 des Endoskops 10, da die Fluoreszenz wieder im Wellenbereich des sichtbaren Lichts liegt. Auf diese Weise können Annotationen zu einer Tumoraktivität und ferner einem Blutfluss in dem 3D-Modell ergänzt werden, um dem Chirurgen zeitaktuelle, für den Eingriff relevante Informationen zur Verfügung zu stellen.

In einer Speichereinheit 30 des Kavitätsmodellierungssystems 1 sind präoperative dreidimensionale Aufnahmen gespeichert, vorliegend MRT-Aufnahmen die zumindest den auch den Eingriffsbereich mit einem zu resezierenden Tumor umfassen. Ferner weist das Kavitätsmodellierungssystem 1 eine Vergleichseinheit 32 auf, die dafür angepasst ist, das intraoperativ erstellte 3D-Modell 2 der Innenoberfläche 18 der Kavität K mit einem dreidimensionalen Außenoberflächenmodell 34 des zu entfernenden Tumors der präoperativen Aufnahme zu vergleichen. Es wird also ein intrakorporales Innenoberflächenmodell mit einem präoperativen Außenoberflächenmodell mittels der Vergleichseinheit 32 verglichen und eine Ansicht dieses Vergleichs wird dann über die Darstellungsvorrichtung (20) auszugeben. Vorliegend wird eine Überlagerungsdarstellung von dem intraoperativen 3D-Modell 2 und dem präoperativen dreidimensionalen Oberflächenmodell 34 ausgegeben, um dem Chirurgen die Bereiche der Unter- bzw. Überresektion anzuzeigen.

Auch kann das Kavitätsmodellierungssystem 1 über die Darstellungsvorrichtung 20 zeitaktuell eine Ansicht des 3D-Modells 2 mit den bereits erfassten Bereichen 36 mit den intraoperativen Aufnahmen 8, 8a, 8b anzeigen und ferner mit den verbleibenden noch nicht erfassten (Oberflächen-)Bereichen 38 anzeigen, die für einen vollständigen Scan der Kavität K noch zu erfassen sind. Bei der manuellen Führung der des Endoskops 10 wird zudem über die Darstellungsvorrichtung 20 eine Anleitung für eine Erfassung der noch zu erfassenden Bereichen an den Chirurgen angezeigt in Form von Pfeilen 40, welche in Form von geraden Pfeilen eine Translationsrichtung und in Form von Drehpfeilen eine Rotationsrichtung vorgeben, ähnlich eines Navigationssystems in einem Auto, um dem Chirurgen eine intuitive, vollständige Erfassungsmodalität der Kavität K bereitzustellen.

Fig. 2 zeigt eine detaillierte Längsschnittansicht durch ein Gehirn des Patienten P, wobei in eine Kavität ein 2D-Endoskop 10 eines Kavitätsmodellierungssystems 1 einer weiteren bevorzugten Ausführungsform eingeführt ist, um die intrakorporalen Aufnahmen 8 zu erstellen und aus diesen Aufnahmen dann das 3D-Modell 2 zu generieren. Man kann gut erkennen, dass das starre Endoskop 10 durch Bewegungen in axialer Richtung als auch Rotationen die gesamte Kavität nach und nach erfassen kann und die 3D-Modellierungseinheit 16 auf Basis der nach und nach hinzugefügten Aufnahmen schließlich die gesamte Kavität K in Form des 3D-Modells 2 nachbilden kann, und zwar intraoperativ. So kann der Chirurg direkt im Operationssaal seinen Erfolg überprüfen.

Das Endoskops 10 kann als 2D-Endoskop mit zweidimensionaler Aufnahme 8 oder als 3D-Endoskop mit dreidimensionaler Aufnahme 8 ausgebildet sein. Die optische Achse 46 verläuft schräg, insbesondere quer zu einer Längsachse 48 des Endoskops 10 ausgerichtet in einem Winkel von 60° zu der Längsachse 48 des Endoskops 10. Damit steht sozusagen die optische Achse 46 in eine radiale Außenseite bzw. Außenoberfläche 50 eines Endoskopschafts 52 ein, Insbesondere kann das Endoskop 10 eine Weitwinkelkamera an seiner radialen Außenseite 50 aufweisen, die einen Sehwinkel von über 60° erfasst, um mittels Rotation die Innenoberfläche 18 der Kavität K zu erfassen.

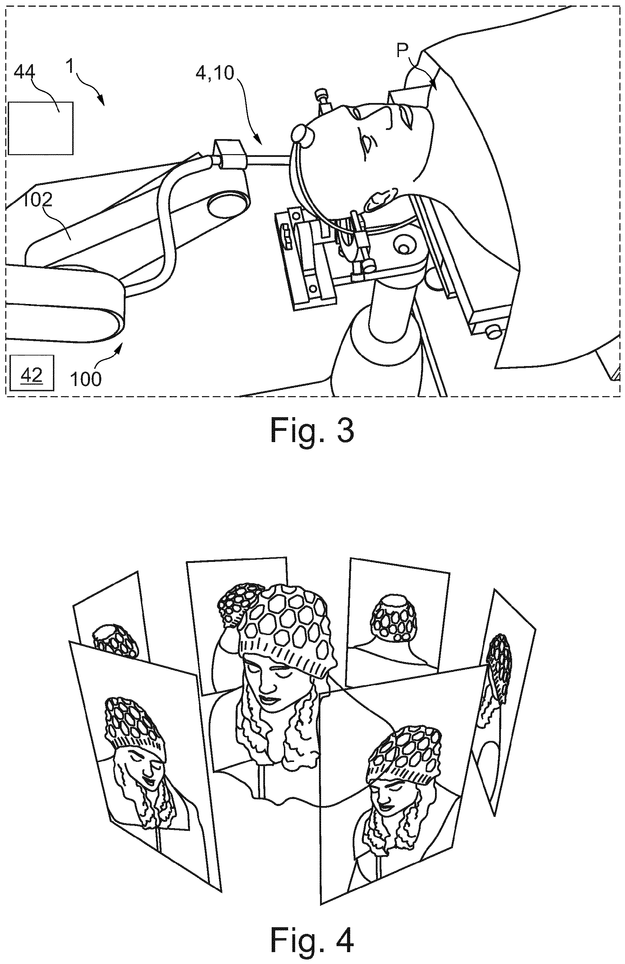

Fig. 3 zeigt, im Gegensatz zu der ersten Ausführungsform, ein robotergestütztes Kavitätsmodellierungssystem 1 gemäß einer weitere, dritten bevorzugten Ausführungsform der vorliegenden Offenbarung. Hierbei wird das Visualisierungseinheit 4 in Form des starren Endoskops 10 nur durch einen Roboter 100 durch dessen Roboterarm 102 geführt. Das Endoskop 10 ist dabei als Endeffektor an dem Roboterarm 102 angebunden und kann im Raum bewegt werden. Insbesondere kann damit eine Position und eine Orientierung des distalen Aufnahmekopfs 6 gesteuert werden. Eine Steuereinheit 42 des Kavitätsmodellierungssystems 1 ist dafür angepasst den Roboter 100 und damit die Lage des Aufnahmekopfs 6 zu steuern und für eine Erfassung der Kavität K diese automatisch abzufahren, um die gesamte Innenoberfläche 18 der Kavität K zu erfassen. In einer durch den Chirurgen auswählbaren Steuerungsmodalität ist die Steuereinheit 42 dafür angepasst, die Position des Aufnahmekopfs 6 über drei Positionsparameter und die Orientierung des Aufnahmekopfs 6 über drei Orientierungsparametern zu steuern. Hierbei ist eine Teilmenge der Parameter, nämlich die der Orientierungsparameter für eine Rotation des Aufnahmekopfs 6 der manuellen Steuerung zugeordnet und eine axiale, translatorische Bewegung über die Positionsparameter der automatischen Steuerung zugeordnet. Über eine Eingabeeinheit 44, hier ein Touchdisplay, kann der Nutzer dann seine Parameter einer Rotation steuern.

Fig. 4 zeigt zum Verständnis schematisch den Vorgang zur Erstellung eines 3D-Modells auf Basis von 2D-Bildern, was analog auf eine Kavität angewendet werden kann. Konkret werden in unterschiedlichen Richtungen zweidimensionale Aufnahmen des Objekts erstellt, aus diesen kann dann das 3D-Modell rückberechnet werden.

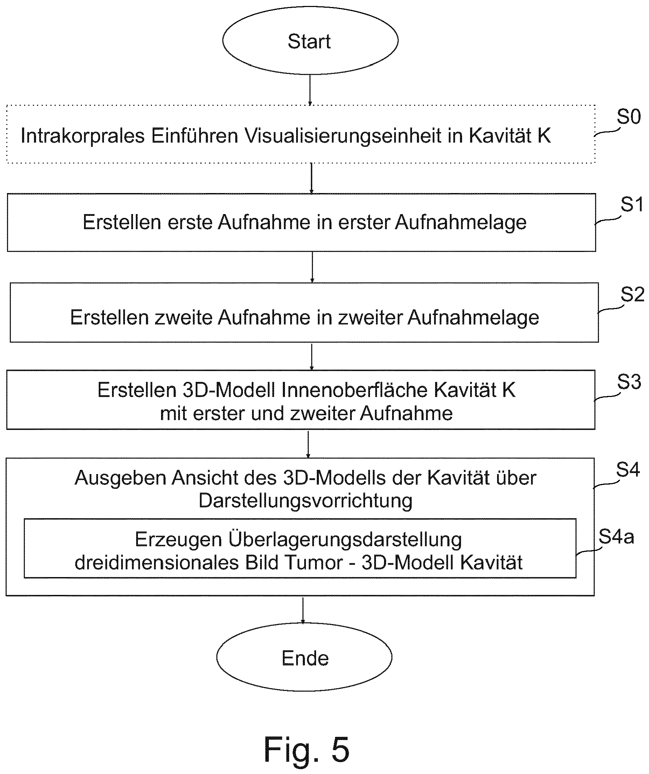

Fig. 5 zeigt ein Kavitätsmodellierungsverfahren gemäß einer bevorzugten Ausführungsform. In einem optionalen Schritt S0 erfolgt ein intrakorporales Einführen einer Visualisierungseinheit 4 mit einem endständigen Aufnahmekopf 6 in Form eines Endoskops 10 mit einem distalen optischen System 12 und einem nachgeschalteten Aufnahmesensor 14 in eine Kavität K des Patienten P zur Erstellung einer intrakorporalen Aufnahme 8 eines Teilbereichs der Kavität K.

In einem ersten Schritt S1 wird eine erste Aufnahme 8a durch das Visualisierungssystem 4 in einer ersten Aufnahmelage erstellt.

In einem zweiten Schritt S2 wird zumindest eine zweite Aufnahme 8b, vorliegend sogar eine Vielzahl an weiteren Aufnahmen 8b, durch das Visualisierungssystem 4 in einer zweiten Aufnahmelage, die unterschiedlich zu der ersten Aufnahmelage ist erstellt und digital bereitgestellt.

In Schritt S3 wird ein 3D-Modells 2 einer Innenoberfläche 18 der Kavität K erstellt mit Ergänzen und Anpassen um die bereitgestellte erste Aufnahme 8a in der ersten Aufnahmelage und um zumindest die weiteren Aufnahmen 8b in den weiteren Aufnahmelagen.

Schließlich erfolgt in Schritt S4 ein Ausgeben einer Ansicht des erstellten 3D-Modells 2 der Kavität K über eine visuelle Darstellungsvorrichtung 20 in Form eines Displays, um einem medizinischen Fachpersonal wie einem Chirurgen eine zeitaktuelle intraoperative Visualisierung der Kavität K bereitzustellen.

### Bezugszeichenliste

- 1: Kavitätsmodellierungssystem
- 2: 3D-Modell
- 4: Visualisierungseinheit
- 6: Aufnahmekopf
- 8: Aufnahme
- 8a: erste Aufnahme
- 8b: zweite Aufnahme
- 10: Endoskop
- 11: proximales Handhabungssystem
- 12: Optisches System
- 14: Aufnahmesensor
- 16: 3D-Modellierungseinheit
- 18: Innenoberfläche
- 20: Darstellungsvorrichtung
- 21: Tracker
- 22: Trackingsystem
- 23: Navigationskamera
- 24: Fluoreszenzaufnahmeeinheit
- 26: Strahler
- 28: Sensor
- 30: Speichereinheit
- 32: Vergleichseinheit
- 34: dreidimensionales Modell
- 36: Erfasste Bereiche
- 38: Nicht erfasste Bereiche
- 40: Pfeil
- 42: Steuereinheit
- 44: Eingabeeinheit
- 46: optische Achse
- 48: Längsachse
- 50: radiale Außenseite
- 52: Endoskopschaft

- 100: Roboter
- 102: Roboterarm

- K: Kavität
- P: Patient

- 50: optionaler Schritt intrakorporales Einführen Visualisierungseinheit
- S1: Schritt Erstellen erste Aufnahme
- S2: Schritt Erstellen zweite Aufnahme
- S3: Schritt Erstellen 3D-Modell
- S4: Schritt Ausgeben Ansicht 3D-Modell
- S4a: Schritt Ausgeben Überlagerungsdarstellung

## Patentansprüche

1. Kavitätsmodellierungssystem (1) für eine intraoperative Erstellung eines 3D-Modells (2) einer Kavität (K) in einem Patienten (P) bei einem chirurgischen Eingriff mit:
einer Visualisierungseinheit (4) mit einem distalen Aufnahmekopf (6), die dafür angepasst ist intrakorporal in den Patienten (P) eingeführt zu werden und über den distalen Aufnahmekopf (6) eine intrakorporale Aufnahme (8) von zumindest einem Teilbereich der Kavität (K) des Patienten (P) zu erstellen und digital bereitzustellen, insbesondere einem Endoskop (10) mit einem distalen optischen System (12) und einem nachgeschalteten Aufnahmesensor (14) zur Erstellung der intrakorporalen Aufnahme (8),
**gekennzeichnet durch**
eine 3D-Modellierungseinheit (16), die dafür angepasst ist, ein digitales 3D-Modell (2) einer Innenoberfläche (18) der Kavität (K) zu erstellen und dieses um eine bereitgestellte erste Aufnahme (8a) in einer ersten Aufnahmelage und um zumindest eine zweite Aufnahme (8b) in einer zweiten Aufnahmelage zu ergänzen und anzupassen, wobei die 3D-Modellierungseinheit (16) ferner dafür angepasst ist, eine Ansicht des erstellten 3D-Modells (2) der Kavität (K) über eine visuelle Darstellungsvorrichtung (20), wie einem Display oder einer Virtual-Reality-Brille, auszugeben, um einem Nutzer wie etwa einem medizinischen Fachpersonal, eine zeitaktuelle intraoperative Visualisierung der Kavität (K) bereitzustellen, und
einen Roboter (100) mit einem Roboterarm (102), an dem die Visualisierungseinheit (4), insbesondere das Endoskop (10), als Endeffektor angebunden ist, wobei eine Steuereinheit (42) des Kavitätsmodellierungssystems (1) dafür angepasst ist, den Roboter (100) und damit die Lage des Aufnahmekopfs (6) der Visualisierungseinheit (4) zu steuern und insbesondere für eine Erfassung der Kavität (K) diese automatisch abzufahren, insbesondere um die gesamte Innenoberfläche (18) der Kavität (K) zu erfassen.

2. Kavitätsmodellierungssystem (1) nach Anspruch 1, **dadurch gekennzeichnet, dass** das Kavitätsmodellierungssystem (1) ein Trackingsystem (22) aufweist, das dafür angepasst ist, eine Position und Orientierung des Aufnahmekopfs (6) der Visualisierungseinheit (4) im Raum nachzuverfolgen, um so die Aufnahmelage der intrakorporalen Aufnahme (8) zu bestimmen, insbesondere das Trackingsystem (22) ein Navigationssystem mit einer externen Navigationskamera aufweist und/oder ein elektromagnetisches Navigationssystem aufweist und/oder eine an der Visualisierungseinheit (4) angeordnete Inertiale Messeinheit aufweist und/oder das Trackingsystem (22) auf Basis einer Roboterkinematik eines chirurgischen Roboters (100) mit der Visualisierungseinheit (4) als Endeffektor die Lage des Aufnahmekopfs (6) bestimmt.

3. Kavitätsmodellierungssystem (1) nach einem der vorstehenden Ansprüche, **dadurch gekennzeichnet, dass** eine Bewegung der Visualisierungseinheit (4) neben einer automatischen Bewegung durch den Roboterarm (102), insbesondere nach einem automatischen Steuerverfahren zur Erfassung der Kavität, auch manuell durch einen Benutzer durchführbar ist, insbesondere kann der Roboter (100) vollständig vom Benutzer manuell gesteuert werden, um die Innenoberfläche (18) der Kavität (K) zu scannen.

4. Kavitätsmodellierungssystem (1) nach einem der vorstehenden Ansprüche, **dadurch gekennzeichnet, dass** die 3D-Modellierungseinheit (16) dafür angepasst ist mittels einer Bildanalyse auf Basis der ersten Aufnahme (8a) mit der ersten Aufnahmelage und der zumindest zweiten Aufnahme (8b) mit der zweiten Aufnahmelage eine dreidimensionale Innenoberfläche eines Bereichs der Kavität (K) oder der gesamten Kavität (K) zu berechnen.

5. Kavitätsmodellierungssystem (1) nach einem der vorstehenden Ansprüche, **dadurch gekennzeichnet, dass** die Visualisierungseinheit (4), insbesondere das Endoskop (10), eine Fluoreszenzaufnahmeeinheit (24) mit sowohl einem Strahler (26) einer vordefinierten Wellenlänge für eine Anregung aufweist als auch mit einem Sensor (28) für eine Detektion aufweist, wobei insbesondere über den Aufnahmekopf (6) mit Aufnahmesensor (14) eine angeregte Fluoreszenz detektierbar ist, um das 3D-Modell (2) der Innenoberfläche der Kavität (K) um weitere Annotationen, insbesondere zu einer Annotationen zu einer Tumoraktivität und/oder einem Blutfluss zu ergänzen, und zeitaktuelle sowie für den Eingriff relevante Informationen zur Verfügung zu stellen.

6. Kavitätsmodellierungssystem (1) nach einem der vorstehenden Ansprüche, **dadurch gekennzeichnet, dass** in einer Speichereinheit (30) des Kavitätsmodellierungssystems (1) präoperative dreidimensionale Aufnahmen gespeichert sind, insbesondere MRT-Aufnahmen und/oder CT-Aufnahmen, die zumindest den Eingriffsbereich mit einem zu resezierenden Gewebe, insbesondere einem Tumor, umfassen, und das Kavitätsmodellierungssystem (1) ferner eine Vergleichseinheit (32) aufweist, die dafür angepasst ist, das intraoperativ erstellte 3D-Modell (2) der Innenoberfläche (18) der Kavität (K) mit einem dreidimensionalen Außenoberflächenmodell (34) des zu entfernenden Gewebes, insbesondere Tumors, der präoperativen Aufnahme zu vergleichen und den Vergleich über die Darstellungsvorrichtung (20) auszugeben, insbesondere eine Abweichung auszugeben, besonders bevorzugt in Form einer Überlagerungsdarstellung von dem intraoperativen 3D-Modell (2) und dem präoperativen dreidimensionalen Oberflächenmodell (34) und/oder einer Angabe einer prozentualen Abweichung eines Volumens oder einer Form, so dass dem Nutzer Bereiche einer Unter- oder Überresektion angezeigt werden.

7. Kavitätsmodellierungssystem (1) nach Anspruch 6, **dadurch gekennzeichnet, dass** die Vergleichseinheit (32) dafür angepasst ist, eine dreidimensionale Form des intraoperativen 3D-Modells und des präoperativen dreidimensionalen Oberflächenmodells zu vergleichen, insbesondere ein Verhältnis einer Breite zu einer Länge zu einer Höhe zu vergleichen und über die Darstellungsvorrichtung (20) auszugeben, insbesondere hinsichtlich einer Abweichung, um über den Formvergleich bei einer sich durch weiches Gewebe veränderlichen Kavität die Resektion zu veranschaulichen und insbesondere eine korrekte Resektion zu bestätigen.

8. Kavitätsmodellierungssystem (1) nach einem der vorstehenden Ansprüche, **dadurch gekennzeichnet, dass** das Kavitätsmodellierungssystem (1) über die Darstellungsvorrichtung (20) zeitaktuell eine Ansicht des 3D-Modells (2) mit erfassten Bereichen (36) mit den intraoperativen Aufnahmen (8, 8a, 8b) anzeigt und mit den verbleibenden Bereichen (38) anzeigt, die noch nicht erfasst sind und bei einer manuellen Führung der Visualisierungseinheit (4), insbesondere des Endoskops (10), eine Anleitung für eine Erfassung der noch zu erfassenden Bereichen an den Nutzer ausgibt, insbesondere in Form von Pfeilen (40), welche in Form von geraden Pfeilen eine Translationsrichtung und/oder in Form von Drehpfeilen eine Rotationsrichtung vorgeben, um dem Nutzer eine intuitive, vollständige Erfassung der Kavität (K) bereitzustellen.

9. Kavitätsmodellierungssystem (1) nach einem der vorstehenden Ansprüche, **dadurch gekennzeichnet, dass** die Steuereinheit (42) dafür angepasst ist, die Position des Aufnahmekopfs (6) über drei Positionsparametern und die Orientierung des Aufnahmekopfs (6) über drei Orientierungsparametern zu steuern, wobei eine Teilmenge der Parameter der automatischen Steuerung zugeordnet ist und durch die Steuereinheit (42) automatisch ausgeführt wird und eine andere, insbesondere verbleibende, Teilmenge der Parameter der manuellen Steuerung zugeordnet wird und über eine Eingabeeinheit (44) durch den Nutzer steuerbar ist, wobei vorzugsweise eine Rotation des Aufnahmekopfs (6) über die Orientierungsparameter der manuellen Steuerung zugeordnet wird und eine axiale, translatorische Bewegung über die Positionsparameter der automatischen Steuerung zugeordnet wird.

10. Kavitätsmodellierungssystem (1) nach Anspruch 9, **dadurch gekennzeichnet, dass** die Steuereinheit (42) dafür angepasst ist die automatische Steuerung durch den Roboter durchzuführen und bei Bedarf die automatische Steuerung teilweise hinsichtlich einer vordefinierten Teilmenge an Parameter an Freiheitsgraden oder auch vollständig durch die manuelle Steuerung abzulösen, so dass ein automatisches Abfahren der Kavität durch eine manuelle Bewegung überschreibbar und damit übernehmbar ist.

11. Kavitätsmodellierungssystem (1) nach einem der vorstehenden Ansprüche, **dadurch gekennzeichnet, dass** die Visualisierungseinheit (4) in Form eines Endoskops (10), insbesondere eines 2D-Endoskops mit zweidimensionaler Aufnahme (8) oder eines 3D-Endoskops mit dreidimensionaler Aufnahme (8), ausgebildet ist und eine Kamera aufweist, dessen optische Achse (46) insbesondere schräg zu einer Längsachse (48) des Endoskops (10) ausgerichtet ist, insbesondere in einem Winkel von 60° zu der Längsachse des Endoskops (10) steht und/oder insbesondere die optische Achse (46) in eine radiale Außenseite (50) eines Endoskopschafts (52) steht und das Endoskop (10) vorzugsweise eine Weitwinkelkamera an seiner radialen Außenseite (50) aufweist, die einen Sehwinkel von über 60° erfasst, um mittels Rotation die Innenoberfläche (18) der Kavität (K) zu erfassen.

12. Kavitätsmodellierungsverfahren für eine intraoperative Erstellung eines 3D-Modells (2) einer Kavität (K) in einem Patienten (P) bei einem chirurgischen Eingriff, insbesondere bei einer Gehirnoperation mit Tumorentfernung, **gekennzeichnet durch** die Schritte:
Vorzugsweise intrakorporales Einführen (S0) einer Visualisierungseinheit (4) mit einem endständigen Aufnahmekopf (6) in eine Kavität (K) des Patienten (P), insbesondere eines Endoskops (10) mit einem optischen System (12) und einem nachgeschalteten Aufnahmesensor (14) zur Erstellung einer intrakorporalen Aufnahme (8) eines Teilbereichs der Kavität (K);
Erstellen (S1) einer ersten Aufnahme (8a) durch das Visualisierungssystem (4) in einer ersten Aufnahmelage;
Erstellen (S2) zumindest einer zweiten Aufnahme (8b) durch das Visualisierungssystem (4) in einer zweiten Aufnahmelage, die unterschiedlich zu der ersten Aufnahmelage ist, mittels eines Roboters (100) mit einem Roboterarm (102), an dem die Visualisierungseinheit (4), insbesondere das Endoskop (10), als Endeffektor angebunden ist, und mittels einer Steuereinheit (42), die dafür angepasst ist, den Roboter (100) und damit die Lage des Aufnahmekopfs (6) der Visualisierungseinheit (4) zu steuern;
Erstellen (S3) eines 3D-Modells (2) einer Innenoberfläche (18) der Kavität (K) mit Ergänzen und Anpassen um die bereitgestellte erste Aufnahme (8a) in der ersten Aufnahmelage und um zumindest eine zweite Aufnahme (8b) in der zweiten Aufnahmelage; und
Ausgeben (S4) einer Ansicht des erstellten 3D-Modells (2) der Kavität (K) über eine visuelle Darstellungsvorrichtung (20), um einem Nutzer wie etwa einem medizinischen Fachpersonal, eine zeitaktuelle intraoperative Visualisierung der Kavität (K) bereitzustellen.

13. Kavitätsmodellierungsverfahren nach Anspruch 12, **dadurch gekennzeichnet, dass** das Kavitätsmodellierungsverfahren ferner die Schritte aufweist:
Vergleichen (S5) einer Form eines präoperativen dreidimensionalen Oberflächenmodells (34) mit dem 3D-Modell (2) und
Ausgeben (S4a), durch die Darstellungsvorrichtung (20), einer, insbesondere einander angepassten skalierten, Überlagerungsdarstellung, insbesondere einer jeweils teiltransparenten Ansicht, des intraoperativen 3D-Modells (2) der Innenoberfläche (18) der Kavität (K) und des präoperativen dreidimensionalen Oberflächenmodells (34), um dem Nutzer die aktuelle Resektion zu veranschaulichen

14. Computerlesbares Speichermedium, umfassend Befehle, die, bei einer Ausführung durch einen Computer, diesen veranlassen, die Verfahrensschritte des Kavitätsmodellierungsverfahrens nach Anspruch 12 oder 13 auszuführen.

15. Computerprogramm umfassend Befehle, die, bei einer Ausführung durch einen Computer, diesen veranlassen, die Verfahrensschritte des Kavitätsmodellierungsverfahrens nach Anspruch 12 oder 13 auszuführen.
